Europäisches Patentamt

European Patent Office  (11) Publication number: **0 215 757**

Office européen des brevets  **B1**

(12) **EUROPEAN PATENT SPECIFICATION**

(45) Date of publication of patent specification: **01.08.90**  (51) Int. Cl.⁵: **A 61 B 6/14**

(21) Application number: **86850287.3**

(22) Date of filing: **02.09.86**

(54) Control system of an X-ray apparatus for panoramic tomography.

(30) Priority: **13.09.85 FI 853524**

(43) Date of publication of application:
**25.03.87 Bulletin 87/13**

(45) Publication of the grant of the patent:
**01.08.90 Bulletin 90/31**

(84) Designated Contracting States:
**AT BE CH DE FR GB IT LI LU NL SE**

(56) References cited:
**EP-A-0 035 307**
**WO-A-85/03423**
**AT-B- 377 908**
**DE-A-3 041 372**
**DE-A-3 304 271**
**DE-A-3 503 465**
**FR-A-2 324 023**
**US-A-4 039 837**

**PATENT ABSTRACTS OF JAPAN, vol. 1, no. 58,
6th June 1977, page 101 E 77; & JP-A-52 2391
(YOICHI SHINOHARA) 10-01-1977**

(73) Proprietor: **PLANMECA OY
Mekaanikonkatu 5
SF-00810 Helsinki (FI)**

(72) Inventor: **Virta, Arto
Pohjolantie 15
SF-01260 Vantaa (FI)**
Inventor: **Strömmer, Pekka
Ilmakuja 5 F 49
SF-02210 Espoo (FI)**

(74) Representative: **Onn, Thorsten et al
AB STOCKHOLMS PATENTBYRA Box 3129
S-103 62 Stockholm (SE)**

Courier Press, Leamington Spa, England.

## Description

The present invention relates to a panoramic dental tomography X-ray apparatus, such as for obtaining images of a dental arch comprising: an arm mounted for rotation in a horizontal plane around a vertical axis (O2); means for rotating said arm including a rotary motion motor; an X-ray generator having an X-ray tube with a tube head, said tube head being mounted at one end of said arm for rotation therewith; a cartridge containing X-ray film and a film transport motor mounted at the other end to said arm for rotation therewith; and means for controlling speeds of said motors.

The design and operation principle of most panoramic X-ray apparatuses for dental photography are such that the X-ray beam is turned around the patient's head in such a way that the dental arch will be photographed as a flat picture on moving film.

In order to get a sharp picture of the object being photographed and to make the structures in front of the object and behind it invisible by "fogging" them out of focus, the transverse velocity of the film with respect to the ray beam must be equal to the sweep velocity of the ray beam in the object multiplied with the magnification ratio. The magnification is determined by the ratio of the distance between the focus and the film to the distance between the focus and the object.

The thickness of the layer being photographed sharply is directly proportional to the distance of the instantaneous center of rotation from the film plane, and inversely proportional to the magnification and to the width of the ray beam. From the point of view of how the object will be represented important is only how the focus, the object and the film plane are located with regard to each other. The instantaneous center of rotation has significance only through the sweep velocity. On account of what has been said above it is possible to form a basic equation of panoramic photography:

$$v_1/v_0 = L_1/L_0,$$

$$v_0 = r,$$

where

$L_0$ = the distance from the focus F to the point being photographed at a given moment,

$L_1$ = the distance from the focus F to the film plane, the angular velocity of the rotary movement around the center of rotation

r = the distance of the point being photographed from the instantaneous center of rotation,

$v_1$ = the velocity of an image point on the picture plane or the film plane.

In order to create an ortogonal fluoroscopic photograph of the teeth and the jaw, typical to panoramic topography X-ray equipment, it must be possible to generate a thin X-ray beam penetrating through the patient, and it must be possible to control the anode current and the anode voltage of the tube producing the X-ray beam according to the properties of the patient while the X-ray beam is rotated around the patient along such a path that the X-ray beam meets the jaw and the teeth as perpendicularly as possible. The film and the film cartridge, located on the opposite side of the patient with respect to the X-ray tube, must also be moved with such a speed that the desired layer of the patient's chinbone and dental system are represented on the film sharply and according to the above provisions.

In the earlier types of the Prior Art the anode voltage and anode current, which remain the same during the whole exposure, are set manually at the X-ray generator, and a motor takes care of said rotary movement, which motor also provides the film transport motion by means of a fixed transmission. The layer being represented sharply is defined by the gear ratio of the mechanism which transmits the motion of the motor to the film cartridge.

In the more recent types of the Prior Art there is a separate motor for the film cartridge motion; usually this motor is of constant-speed type, and the gear ratio of the film cartridge is mechanically changed by means of forced control provided by the rotary movement.

A drawback of these known control systems based on mechanically interconnected motion speeds is that the ratio of said motion speeds is mechanically fixed (forced control) and cannot be changed in the different stages of the exposure, therefore only one type of jaw profile can be photographed sharply with these devices. As known human jaws vary a lot, and therefore it has been necessary to make compromises in the known devices. The known devices have been tuned up to make sharp photograph of a so called average jaw profile in order that different jaws would be photographed at least satisfactorily.

For eliminating the drawbacks mentioned above, in some newer devices there is a separate film cartridge transport motor, whose speed can be varied as before with control provided by the rotary motion but also separately so that the layer being photographed sharply can when desired be changed within certain limits thus aiming at getting as good panoramic photograph as possible of each jaw shape.

In the last-mentioned known equipment, in which the rotary motion and the film cartridge movement have their own motors, the rotary motion motor is a constant-speed motor; in consequence it has been possible to change the layer being photographed sharply only by changing the speed of the film cartridge. This procedure has the inevitable consequence that the exposing time will change directly proportionally to the film cartridge transport speed, and the picture will not be evenly exposed; instead, it will have lighter and darker spots depending on to what direction the sharply-represented layer has been corrected with respect to the basic shape determined by the mechanical system. Similarly, in these known systems with two separate motors it has not been possible to make the necessary correction in order to compensate the absorption of radiation caused by the cervical spine, because always at least one of the motors is of the constant-speed type. Therefore it has been necessary to make the required compensations by changing the intensity of the X-ray beam, but as the milli-ampere-regulation with which the intensity is controlled is too slow due to proporties of the glow filament of the X-ray tube, one must change the intensity by changing the anode voltage of the X-ray tube. Depending on the properties of the patient and the used ampilfication boards, the maximum contrast is achieved with a certain anode voltage, deviating from this voltage results in worse contrast, and with known equipment the only choice has been to content oneself with this.

Accordingly, it is an object of this invention to provide a panoramic dental tomography equipment, in which said drawbacks have been eliminated; when required, the invention also offers more advantageous features which have not at all been available with the known equipment.

According to EP—A2—0035307 control signals for motor drives are derived from the contour of the dentition by means of a plate which is covered by contacts on which the patient exerts a pressure by biting, so that the contacts whose position corresponds to the shape of the dentition are actuated. Therefrom, the contour of the dentition is determined by an arithmetic unit which supplies the control signals for the drive motors. The reference thus starts from the assumption that jaws of any shape can be photographed without restriction. Therefore, the reference requires, in addition to a film transport motor and a rotary motion motor of a tube head, two separate motors and complicated x-y mechanisms for movement in the direction of the center of rotation x and y.

According to the invention said separate motors and complicated mechanisms will not be required. Thus, the new X-ray apparatus as defined in the description is constructed in such a way that the center of rotation of said arm is smoothly guided along a curved, mechanically predetermined and fixed path, which path is the same irrespective of the shape and size of the jaw being photographed, that in the front area of the dental arch the said arm rotates (sector c) around said fixed vertical axis (O2), and before and after said fixed rotation (sector c) the center of rotation of said arm (u) smoothly moves along said curved path (sector d) to a predetermined vertical axis (O3), and in the end areas of the dental arch the vertical axis of rotation further draws away along said curved path (sector e) from the center axis $(a_0)$ of the jawbone (j) under photography, that said control system means is arranged to adjust the speed of said rotary motion motor and arranged to maintain the speed of said film transport motor substantially constant thereby driving the film of constant speed, so that the location of an arch layer being photographed is changed only by varying the speed of the rotary motion motor during an exposure sequence and that by guidance of operator control data and a working program stored in said control system means, dental arches of varying sizes and shapes are photographed by said panoramic tomography X-ray apparatus both sharply and within a suitable exposure time.

In an apparatus in accordance with the invention the speeds of both the rotary motion motor and the film cassette transport motor can be adjusted separately, and the control system also controls the kilovolt and milliampere values during the exposure, preferably continuously. It is also possible to use an automatic primary blind which is adjusted or changed by the same control system.

The invention will now be described in detail with reference to some exemplary feasible embodiments illustrated in the figures of the accompanying drawings, with no intention to restrict the invention to the details of these embodiments.

Figure 1 is for background information and shows a projection which can be mastered with a control system in accordance with the invention.

Figure 2 shows a central vertical section of a device and mechanism which is controlled with a control system in accordance with the invention.

Figure 3 is a block diagram of a control system in accordance with the invention.

Figure 4 shows examples of dental arches of different sizes which can be photographed sharply thanks to the control system in accordance with the invention.

Figure 5 shows in the same way as Figure 4 examples of dental arches of varying shapes, which can be photographed sharply thanks to the control system in accordance with the invention.

Figure 6 shows examples of some advantageous speed distributions between the rotary motion motor and the film transport motor during the exposure sequence.

Figure 7 shows a favourable example of how the anode voltage of the X-ray tube is controlled with a control system in accordance with the invention.

Figure 8 illustrates how a primary blind controlled with a system in accordance with the invention can be embodied.

Figure 9 illustrates another example of the controlled blind.

Figure 10 illustrates a third example of the controlled blind.

Figure 11 is a schematic illustration of a control system in accordance with the invention for taking cephalostatic photographs.

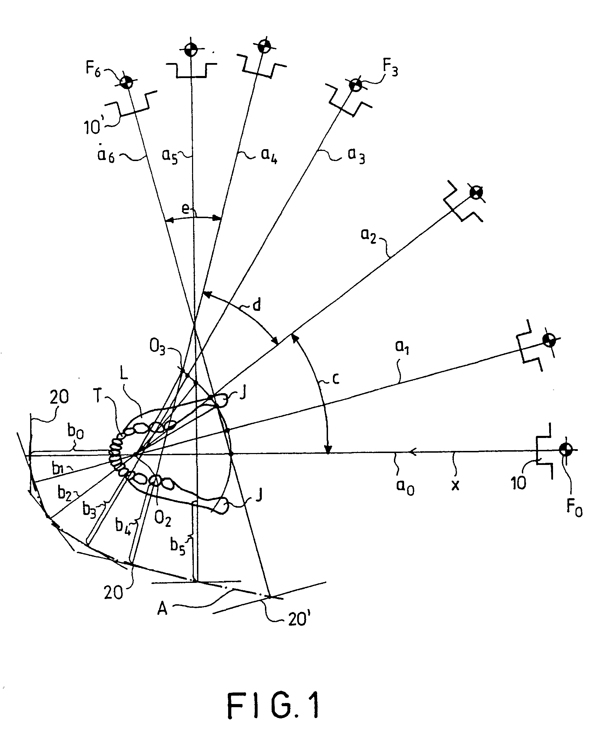

Figure 1 illustrates the photographing geometry and projection accomplished with the apparatus in accordance with the invention. The X-ray tube is marked with reference number 10, its focus in different positions with references $F_0$ to $F_6$. The X-ray tube 10 transmits an X-ray beam X through the teeth T and the jawbone L onto a film 20 along the line a. In Figure 1, the passage of the X-ray X is shown in seven different positions

$a_0$ to $a_6$. In an end position, the X-ray tube is marked with 10', the film with 20' and the focus of the tube with $F_6$. The joints of the jawbone L are marked with J.

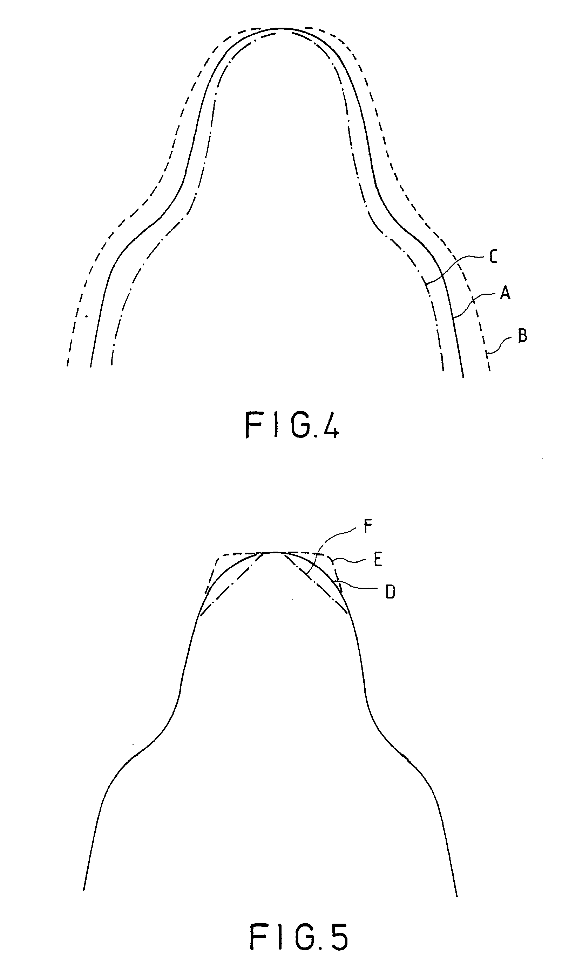

In the front area of the dental arch, which is represented by the space between the rays $a_0$ and $a_1$ (sector c), the arm 11, carrying the X-ray tube 10 on its one end and the cartridge 20 on its other end, rotates in the horizontal plane around the vertical axis $O_2$. Between the rays $a_2$ and $a_4$ (sector d), the center of rotation smoothly moves along a curved path to the vertical axis $O_3$, after which the vertical axis further draws away from the center axis $a_0$. With the geometry illustrated in Figure 1, the ortogonality of the representation comes true with good accuracy besides in the front dental arch area and in the side areas also in the rear part of the jawbone L up to the joints J. Magnification will also be constant in the whole image field; this is proved by for instance the fact that the distance $b_0$ to $b_5$ of the film from the photographed layer is precisely enough constant over the whole path. As the distance $b_0$ to $b_5$ is constant, the film 20 can be brought closer to the patient P (Figure 5) in order to reduce the magnification ratio without a risk of the film cartridge hitting the patient.

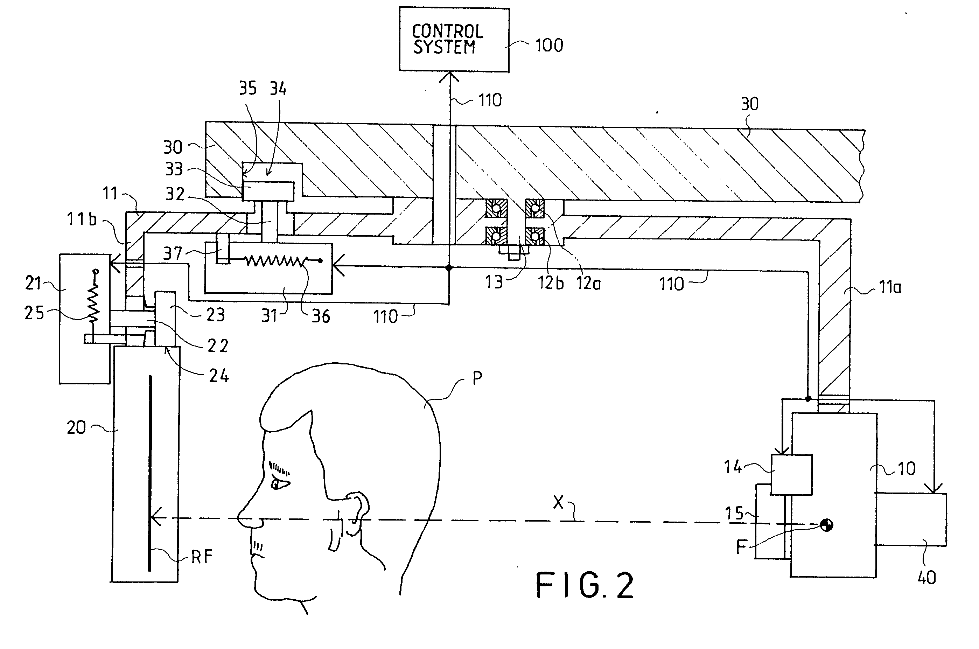

Figure 2 shows only schematically the mechanism with which the arm that connects the X-ray tube 10 and the film cartridge 20 is rotated in the horizontal plane around axis 13 and around other rotary axes located near it for instance according to the principle described in association with Figure 1. As to the details of this mechanism we refer to the applicant's earlier Finnish patent application No. 852208 (filed in May 31, 1985). A control system in accordance with the invention can be feasibly used in association with this mechanism, although the invention is by no means restricted to be used only in association with a mechanism in accordance with said Finnish application.

The apparatus comprises a frame 30, which can vertically be moved to a suitable working height on a base structure. On the frame 30 such a horizontally rotating arm is suspended by means of for instance a mechanism in accordance with the Finnish patent application No. 852208, which arm, at one end, has the X-ray tube 10 supported by a vertical part 11a, and at the other end, supported by a vertical part 11b, has a film cartridge 20 containing the X-ray film RF, the transport mechanism, and other devices in accordance with the invention. The rotating arm 11 is suspended by means of bearings 12a and 12b to be rotated around a vertical shaft. The vertical shaft is not fixed but rotates horizontally with respect to the fixed frame 30 during the exposure. The arm 11 is borne by for instance two opposite bevelled ball bearings 12a, 12b.

The arm 11 is rotated around the vertical shafts by a motor 31, whose axle has the drive wheel 33 at its end. Said drive wheel 33 is pressed by means of a spring 36 fastened to a pin 37 against the vertical side 35 of the drive groove 34. The shape of the groove 34 is substantially similar to the main shape of the jawbone and the dental arch according to the details described in the Finnish patent application No. 852208. The film cartridge is moved in the direction of the film plane with a film transport motor 21, which, at the end of its shaft, has a drive wheel 23, which has a driving contact with the flat top side 24 of the film cartridge or similar. Motors 21 and 31 can suitably be for instance stepping motors or synchronous motors, which are controlled by means of the control system 100 in accordance with the invention. The control system 100 also controls the drive motor 14 of the primary blind 15 and the X-ray generator 40 as is more closely described below.

Figure 2 illustrates in principle the structure of a control system in accordance with the invention, and one possible arrangement of its components in an X-ray apparatus for panoramic tomography. During the exposure an X-ray beam X is directed to the patient P from the focus F of the tube head 10. After passing through the patient the X-ray beam hits the film RF on the cartridge 20 creating onto the film a latent picture of the patient P. During the exposure the tube head 10 and the cartridge holder 20 turn around the patient along a motion path determined by the mechanism so that the motor 31 turns the tube head 10 and the arm 11 supporting the cartridge holder 20 with respect to the suspension 12a, 12b, 13 at a speed determined by the control system. The film cartridge RF is simultaneously moved in its holder 20 with the motor 21 at a speed determined by the control system 100. The system 100 has a keyboard and a dispaly for entering and monitoring control instructions, it also has control electronics required by the motors 21 and 31 and the tube head 10. The system 100 is connected to its pheripherals by a cable 110.

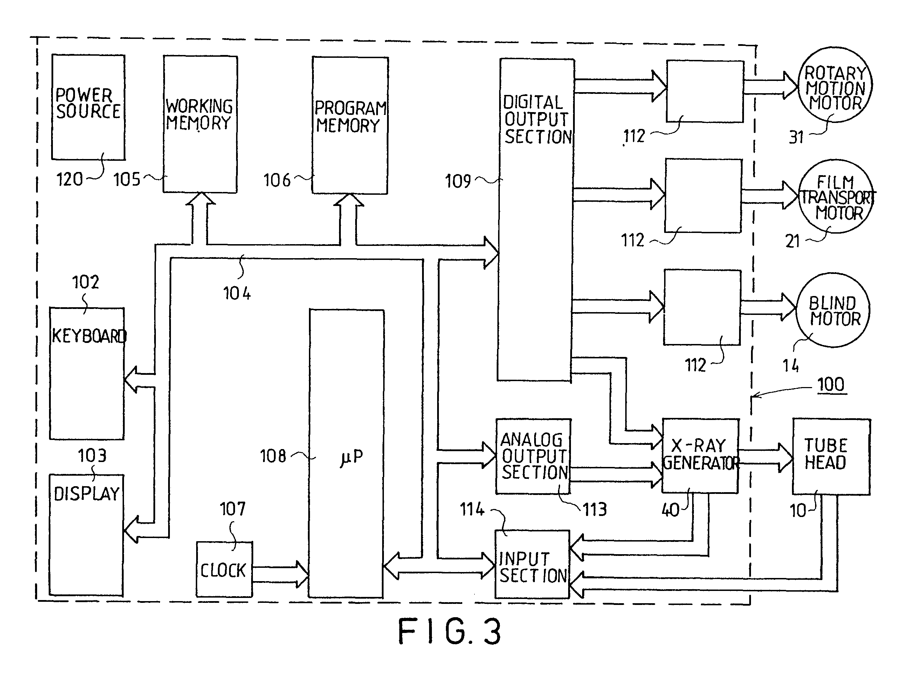

Figure 3 illustrates one possible embodiment of the control system. The functions are controlled by a microprocessor 108 synchronized by a clock oscillator 107. The microprocessor 108 also inherently contains a program memory 106 and a working memory 105. The system is controlled by means of the keyboard 102 and the display 103. A digital output section 109 is connected to the bus 104 of the microprocessor 108, with which the microprocessor controls the rotation directions and speeds of the rotary motion motor 31, the film transport motor 21 and the primary blind 13 by means of control electronic systems 112. The microprocessor controls the X-ray generator 40 besides with the digital output section 109 also with the analog output section 113. The generator feeds the tube head 10, of which, and also of the generator itself, the microprocessor gets status information via the analog/digital input section 114. The different sections get their operating power from the power source 120.

One of the advantages of an apparatus in accordance with the invention is the possibility to photograph jaws of different sizes as shown in Figure 4. In Figure 4 the curve A illustrates a so-called average jaw size, and the mechanism of the apparatus has been designed for this jaw size. Such speeds of the rotary motion motor 31 of the arm 11 and the transport motor 21 of the film cartridge 20 are stored in the control system 100 for each point of the curve A that the layer being photographed sharply exactly conforms with the curve A. However, one feature of the control system is that the given speeds can be changed as desired from the keyboard 102 so that for instance a jaw structure which is larger than average or smaller than average (children) will be photographed sharply and also with a constant exposing time.

In addition that the size definition of the jaw being photographed can be varied the definition of the shape of the jaw can also be varied. An example of this are curves D, E and F in Figure 5, of which the curve D illustrates the shape of an average jaw, the curve E illustrates such a jaw in which the location of the dental system is "box-like" and the curve F illustrates such a jaw in which the dental system is "boat-like". Regardless of the shape and size of the jaw being photographed the control system 100 always produces a sharp picture that is evenly exposed from edge to edge.

For instance variations shown in Figures 4 and 5 are carried out by changing the speed relations of the rotary motion motor 21 and the film transport motor 31 on the basis of the interdependence equations determined by mechanical motion paths. It can be said that in each point of the motion path the speeds of said motors 21, 31 must be in a certain speed relation (1) depending on the point of the motion path in order that the desired layer be sharply photographed on the film RF.

$$v_f = l \times r_0 / [(l - r_f + r_0) \times r_d] \times v_d \qquad (1)$$

where

$v_f$=film transport speed
$v_d$=drive speed of rotary motion,
l=distance from the focus to the film plane,
$r_0$=distance of the layer being photographed from the instantaneous center of rotation,
$r_f$=distance of the film plane from the instantaneous center of rotation,
$r_d$=distance of the rotation drive point from the instantaneous center of rotation.

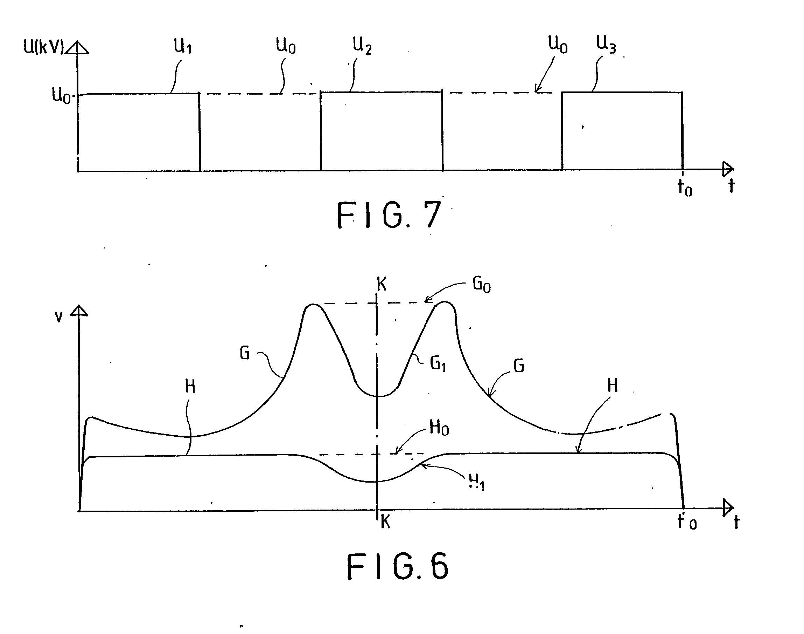

If the speed ratio $v_f/v_d$ is changed by correcting the speed of the film cartridge 20, the place of the layer being photographed and also the exposing time will be changed. If the ratio is changed by changing the speed of the rotary motion motor 31, the place of the layer being photographed will change again, but the exposing time will remain constant, as the film RF moves at even speed across the X-ray beam. It is a result of equation (1) that if the speeds $v_f$ and $v_d$ of motors 21, 31 are changed in the same proportion, the layer being photographed sharply will stay in place but the exposing time will change. Curves C and H in Figure 6 show in principle in association with one mechanical embodiment how the drive speed $v_d$

of the rotary motion motor 31 is a function of time t during the exposure sequence $t_0$ (curve G) and how the drive speed $v_f$ of the transport motor 21 of the film cartridge 20 is a function of time t (curve H). When following the transport speed of the film RF (along the dotted line $H_0$), it can be seen to remain unchanged during the whole exposure sequence $t_0$ while the layer corrections are carried out by varying the speed $v_d$ of the rotary motion motor 31, whereat the exposing time will be constant over the whole area of film RF. However when photographing the front part of the dental arch it is necessary to pass the X-ray beam through the cervical spine, the exposing time that suits to other areas will no more be sufficient but must be locally increased. The curved sections $G_1$ and $H_1$ in the middle of the curves G and H illustrate how the speeds $v_d$ and $v_f$ are slowed down in these areas in order to increase the exposing time, however in constant mutual relation in order to keep the layer being photographed sharply in place. The curves G and H are symmetrical with regard to the center line KK.

Besides that the control system 100 separately controls the speeds of the motors 21 and 31, whereat it is possible to photograph sharply and with constant exposing time a jaw profile of almost any shape or size, the control system 100 will preferably at the same time control the X-ray generator 40. As said above, the intention is to carry out the normal X-ray photographing with constant voltages and constant currents of the X-ray generator 40. The invention makes it possible to also photograph desired areas with different anode voltages if required for instance for improving the contrast; it is also possible to omit certain areas of the dental arch altogether from the X-ray photograph. In all cases the exposing time can be kept constant by changing the speeds $v_d$ and $v_f$ of the motors 21 and 31, even though the anode voltage of the X-ray tube is changed during the exposure sequence $t_0$.

One example is the case shown in Figure 7, in this is case a photograph is required only of both condyles and also of the front teeth. In order to minimize the radiation load on the patient the check areas need not be photographed at all and the radiation may in these areas be interrupted altogether. In the figure the curve $U_1$ illustrates the anode voltage given to the X-ray tube; it is controlled from the keyboard 102 and dropped to zero after passing the condyle area. Similarly the curve $U_2$ illustrates the anode voltage given at the front area of the dental arch; this anode voltage is interrupted for the time of the next check to be switched on again in the next condyle area (curve $U_3$). In normal photography the anode voltage will remain the same during the whole exposure (voltage $U_0$).

In order to maintain the radiation load on the patient as low as possible in all situations, it is not always necessary to photograph the patient on the height of the whole picture. For this purpose the system has an option for automatic changing of the primary blind so that it is possible with a suitable blind to horizontally crop off part of the radiation. By combining this feature with the above described horizontal outlining of the image fields only very small squares of the patient may be photographed. An example of this is photographing salivary glands or maxillary sinuses, in which, when operating in accordance with the invention, the radiation load on the patient will be reduced to about 20% when compared with a full-size panoramic X-ray photograph. This is particularly significant when the patient must attend long-term, regular observation and the allowed radiation load dose is in danger of being exceeded. Similarly when photographing childs the top section of the radiation beam can be cropped off with a separate primary blind thus eliminating unnecessary radiation in the brain area.

In the Prior Art an automatic primary-blind change option has not been known. When carried out in accordance with the invention and combined with the rest of the control system the mistakes will be avoided and the blind 15 may be changed as required through control by the system 100 during the exposure sequence.

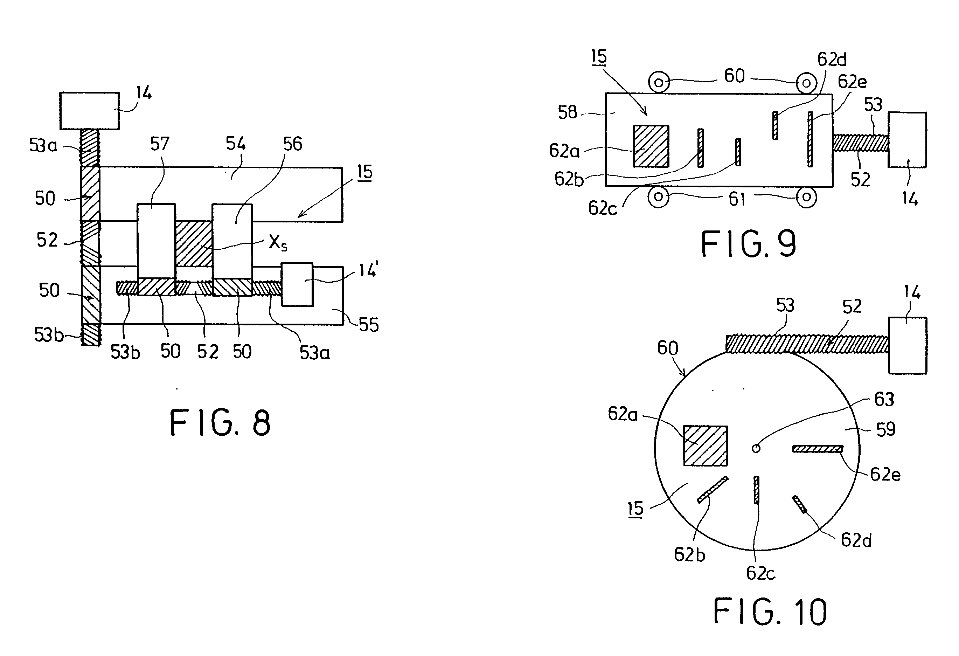

The primary blind is changed to such a way that in the radiation beam X of the X-ray tube, next to the tube head 10, there is a board 15 made of material being able to obstruct radiation, in which board there are openings of the shape of the desired radiation beam X, or the shape of the opening can be changed by a control system. The board 15 is moved by a motor so that an opening having the desired shape will be the primary blind of the radiation beam X, or alternatively the shape of the opening is changed by moving with a motor or motors the radiation-obstructing plates forming the edges of the opening. Different embodiments, which will be described below, are illustrated in Figures 8, 9 and 10.

In Figures 8, 9 and 10 the motors of the blind 15 are marked with 14 and 14', which motors are controlled by a control system 100 in accordance with the invention. As shown in Figure 8, the blind 15 comprises two motors 14 and 14', which drive two screws 52 located perpendicularly to each other. In screws 52 there are threaded sections 53a, 53b with opposite threads, to which the blind boards 54, 55 and 56, 57, edging the perpendicular beam opening $X_s$, are fastened with a threaded part 50. As shown in Figure 9, the motor 14 drives a threaded 53 screw 52 which moves the blind board 58 by means of a threaded part in the blind board 58. In the blind board 58, which is supported by rolls 60, 61 to be able to move horizontally, there is a set of different blind openings 62a, 62b, 62c, 62d and 62e, which may at will be, by means of the motor 14 controlled by the control system 100, moved in front of the X-ray beam X to limit the beam. Figure 10 illustrates such variation of the device in Figure 9 which comprises a round blind board 59 suspended at its center to the frame of the device with a shaft 63. On the outer perimeter of the blind board 59 there is toothing 60, against which the thread 53 of the screw 52 driven by the motor 14 meshes so that by turning the blind board 59 around its center axis 63 under control of the system 100 the suitable one of the blind openings 62a, 62b, 62c, 62d or 62e may be selected.

In some known panoramic tomography X-ray devices there is also an option for taking cephalostatic pictures, which has however usually been inconvenient and required special measures of the user of the equipment such as turning the tube head, manual changing of the primary blind, or for this purpose the apparatus has had for instance a separate tube head with a blind, which makes the apparatus expensive and complicated.

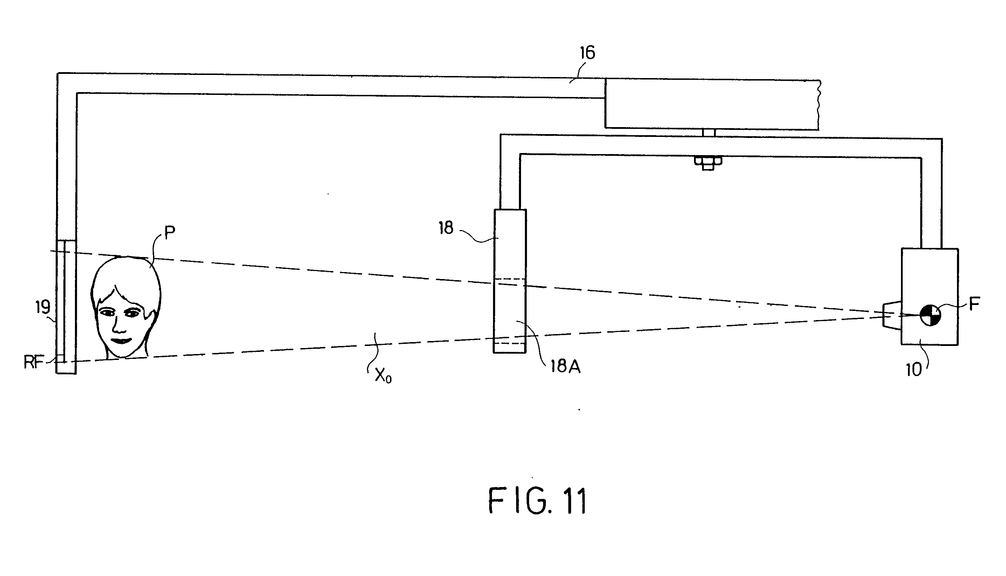

By using the control system 100 in accordance with the invention taking cephalostatic pictures is a simple operation; all that is required is a control command given from the keyboard 102. The principle of how the apparatus in accordance with the invention is used for cephalostatic X-ray photography is illustrated in Figure 11. As it is a feature of the control system 100 to precisely control said motors 21 and 31 and the generator 40, the operation may be carried out for instance in such a way that the a primary blind suitable for cephalostatic photography is substituted for the usual primary blind, the arm carrying tube head 10 and the cartridge holder 20 is driven to such a position with regard to the fixed suspension that the direction of the tube head 10 is correct with regard to the suspension carrying the cephalostat and consequently also with regard to the cephalostat, and by means of the transport motor 21 of the film cartridge 20 the cartridge and other possible fastenings of the cartridge are driven to such a position that the X-ray beam $X_0$ freely passes through an opening 18a formed to the cartridge head 18 through the patient P onto the X-ray film RF, which is in the cartridge 19 fastened to the caphalostat. As the system is able to completely control also the X-ray generator 40, it can be used for an exposure carried out with desired kilovolt/milliampere/second values. Therefore the control system 100 has without any additions a full readiness to act as the control system for cephalostatic photography, only a cephalostate known as such must be added to the panoramic tomography X-ray apparatus.

**Claims**

1. Panoramic dental tomography X-ray apparatus, such as for obtaining images of a dental arch comprising:

an arm (11) mounted for rotation in a horizontal plane around a vertical axis (O2);

means for rotating said arm (11) including a rotary motion motor (31);

an X-ray generator (40) having an X-ray tube with a tube head (10), said tube head (10) being mounted at one end of said arm (11) for rotation therewith;

a cartridge (20) containing X-ray film (RF) and a film transport motor (21) mounted at the other end of said arm (11) for rotation therewith; and

means for controlling the speeds of said motors (21, 31), characterized in

that the center of rotation ($O_2$, $O_3$) of said arm (11) is smoothly guided along a curved, mechanically fixed path, which path is the same irrespective of the shape and size of the jaw being photographed,

that in the front area of the dental arch the said arm rotates (sector c) around said fixed vertical axis ($O_2$), and before and after said fixed rotation (sector c) the center of rotation of said arm (11) smoothly moves along said curved path (sector d) to a predetermined vertical axis ($O_3$), and in the end areas of the dental arch the vertical axis of rotation further draws away along said curved path (sector e) from the center axis ($a_0$) of the jawbone (J) under photography,

that control system means (100) is arranged to adjust the speed ($v_d$) of said rotary motion motor (31) and arranged to maintain the speed ($v_f$) of said film transport motor (21) substantially constant thereby driving the film at constant speed so that the location of an arch layer being photographed is changed only by varying the speed of the rotary motion motor (31) during an exposure sequence, and

that by guidance of operator control data and a working program shored in said control system means, dental arches of varying sizes and shapes are photographed by said panoramic tomography X-ray apparatus both sharply and within a suitable exposure time.

2. Panoramic tomography X-ray apparatus according to claim 1, characterized in that the control system means (100) also controls the aperture of an automatic primary blind (15) and/or the change of apertures (62) of a blind.

3. Panoramic tomography X-ray apparatus according to one of claims 1 or 2, characterized in that the control system means (100) comprises a combination of

a microprocessor (108) comprising the program memory (106) and the working memory (105), equipment (102, 103) for controlling the system, a connecting part connected to the bus (104) of the microprocessor (108), with which connecting part the microprocessor (108) is arranged to control the rotating directions and speed distributions of the rotary motor (31) of the arm (11) of the device and the film transport motor (21).

4. Panoramic tomography X-ray apparatus according to claim 3, characterized in that said connecting part controls, in addition to the motors (21, 31), also the motor (14) of the primary blind (15) via control electronics (112).

5. Panoramic tomography X-ray apparatus according to claim 3 or 4, characterized in that said connecting part of the microprocessor (108) also controls the X-ray generator (40) feeding the tube head (10) of the device, and that the microprocessor (108) is arranged to receive status information from the tube end (10) via the connecting part.

6. Panoramic tomography X-ray apparatus according to one of claims 1 to 5, characterized in that the control system means (100) is programmed to control both the speed distribution (G) of the rotary motor (31) of said arm (11) and the speed distribution (H) of the film transport motor (21) independently of each other.

7. Panoramic tomography X-ray apparatus according to one of claims 1 to 6, characterized in that the normal photographings are carried out with a constant anode voltage and/or a constant anode current of the X-ray generator (40).

8. Panoramic tomography X-ray apparatus according to one of claims 1 to 6, characterized in that by controlling the anode voltage of the X-ray generator (40) only certain areas of the dental arch are photographed.

9. Panoramic tomography X-ray apparatus according to one of claims 1 to 8, characterized in that in order to minimize the radiation load of the patient, the primary blind (14, 15) is, when there is no need to photograph the patient over the full picture height, controlled by the control system means (100).

10. Panoramic tomography X-ray apparatus according to one of claims 1 to 9, characterized in that the system is programmed to photograph only small squares of the patient (P) such as regions of salivary glands and maxillary sinuses.

11. Panoramic tomography X-ray apparatus according to one of claims 1 to 10, characterized in that the control system means (100) is arranged to control the anode current, the anode voltage and/or the exposure time of the X-ray tube in accordance with requirements of cephalostatic photography.

12. Panoramic tomography X-ray apparatus according to claim 11, characterized in that the control system means (100) is arranged to control the adjustment of the tube head (10) with respect to the cephalostat and/or the control system means (100) is adjusted to control the change of blind required by caphalostatic photography.

**Patentansprüche**

1. Zahnärtzliches Panoramatomografie-Röntgengerät, z.B. zum Erhalten von Bildern eines Zahnbogens, bestehend aus:

einem für Drehung in einer horizontalen Ebene um eine vertikale Achse (O2) befestigten Arm (11),

Mitteln zum Drehen genannten Armes (11), einschliesslich eines Drehbewegungsmotors (31), einem Röntgengenerator (40) mit einer Röntgenröhre, deren Röhrenkopf (10) an einem Ende des genannten Armes (11) für Drehung mit demselben befestigt ist,

einer Kassette (20), Röntgenfilm (RF) sowie einen Filmtransportmotor (21) enthaltend, am anderen Ende des genannten Armes (11) für Drehung mit demselben befestigt, und

Mitteln zur Steuerung der Geschwindigkeit der genannten Motoren (21, 31), dadurch gekennzeichnet,

dass das Drehzentrum ($O_2$, $O_3$) des genannten Armes (11) stossfrei längs einer gekrümmten, mechanisch befestigten Bahn geführt ist, die ungeachtet der Form und Grösse des photographierten Kiefers dieselbe ist, dass im Frontbereich des Zahnbogens der genannte Arm um genannte feste vertikale Achse ($O_2$) rotiert (Sektor c), und vor und nach genannter fester Drehung (Sektor c) das Drehzentrum des genannten Armes (11) sich stossfrei längs genannter gekrümmter Bahn (Sektor d) zu einer vorbestimmten vertikalen Achse ($O_3$) bewegt, und in den Endbereichen des Zahnbogens die vertikale Drehachse von der Zentrumachse ($a_0$) des photographierten Kiefers (J) längs genannter gekrümmter Bahn weiter wegzieht (Sektor e),

dass ein Steuersystemmittel (100) angeordnet ist, die Geschwindigkeit ($v_d$) des genannten Drehbewegungsmotors (31) zu verstellen und die Geschwindigkeit ($v_f$) des genannten Filmtransportmotors (21) im wesentlichen konstant zu halten und dabei den Film mit konstanter Geschwindigkeit zu drehen, so dass die Lage einer photographierten Bogenschicht nur durch Änderung der Geschwindigkeit des Drehbewegungsmotors (31) während einer Belichtungsfolge geändert wird,

und dass unter Führung durch Bedienersteuerdaten und einem in genanntem Steuersystemmittel gespeicherten Arbeitsprogramm Zahnbögen von verschiedenen Grössen und Formen durch genanntes Panoramatomografie-Röntgengerät sowohl scharf als auch innerhalb einer geeigneten Belichtungszeit photographiert werden.

2. Panoramatomographie-Röntgengerät nach Anspruch 1, dadurch gekennzeichnet, dass das Steuersystemmittel (100) auch die Öffnung einer automatischen Primärblende (15) und/oder die Änderung der Öffnungen (62) einer Blende steuert.

3. Panoramatomografie-Röntgengerät nach Anspruch 1 oder 2, dadurch gekennzeichnet, dass das Steuersystemmittel (100) eine Kombination enthält, bestehend aus:

einem Mikroprocessor (108), das Programmgedächtnis (106) und das Arbeitegedächtnis (105) enthaelnd,

Ausrüstung (102, 103) zur Steuerung des Systems, einem an den Bus (104) des Mikroprocessors (108) angeschlossenen Verbindungsteil, mit dem der Mikroprocessor (108) angeordnet ist, die Drehrichtungen und Geschwindigkeitsverteilungen des Drehmotors (31) des Armes (11) der Vorrichtung und den Filmtransportmotor (21) zu steuern.

4. Panoramatomografie-Röntgengerät nach Anspruch 3, dadurch gekennzeichnet, dass genanntes Verbindungsteil ausser den Motoren (21, 31) auch den Motor (14) der Primärblende (15) über Steuerelektronik (112) steuert.

5. Panoramatomografie-Röntgengerät nach Anspruch 3 oder 4, dadurch gekennzeichnet, dass genanntes Verbindungsteil des Mikroprocessors (108) auch den Röntgengenerator (40) steuert, der den Röhrenkopf (10) der Vorrichtung speist, und dass der Mikroprocessor (108) angeordnet ist, Statusinformation vom Röhrenende (10) über das Verbindungsteil zu erhalten.

6. Panoramatomografie-Röntgengerät nach einem der Ansprüche 1—5, dadurch gekennzeichnet, dass das Steuersystemmittel (100) programmiert ist, sowohl die Geschwindigkeitsverteilung (G) des Drehmotors (31) des genannten Armes (11) als auch die Geschwindigkeitsverteilung (H) des Filmtransportmotors (21) unabhängig voneinander zu steuern.

7. Panoramatomografie-Röntgengerät nach einem der Ansprüche 1—6, dadurch gekennzeichnet, dass die normalen Photographierungen mit einer konstanten Anodenspannung und/oder einem konstanten Anodenstrom des Röntgengenerators (40) ausgeführt werden.

8. Panoramatomografie-Röntgengerät nach einem der Ansprüche 1—6, dadurch gekennzeichnet, dass durch Steuerung der Anodenspannung des Röntgengenerators (40) nur bestimmte Bereiche des Zahnbogens photographiert werden.

9. Panoramatomografie-Röntgengerät nach einem der Ansprüche 1—8, dadurch gekennzeichnet, dass zur Verminderung der Strahlungsbelastung des Patienten auf ein Minimum die Primärblende (14, 15), wenn keine Notwendigkeit des Photographierens des Patienten in voller Bildhöhe besteht, durch das Steuersystemmittel (100) gesteuert wird.

10. Panoramatomografie-Röntgengerät nach einem der Ansprüche 1—9, dadurch gekennzeichnet, dass das System programmiert ist, nur kleine Vierecke des Patienten (P), wie Speicheldrüsen und Oberkieferhöhlen, zu photographieren.

11. Panoramatomografie-Röntgengerät nach einem der Ansprüche 1—10, dadurch gekennzeichnet, dass das Steuersystemmittel (100) angeordnet ist, den Anodenstrom, die Anodenspannung und/oder die Belichtungszeit der Röntgenröhre gemäss den Erfordernissen zephalostatischer Photographie zu steuern.

12. Panoramatomografie-Röntgengerät nach Anspruch 11, dadurch gekennzeichnet, dass das Steuersystemmittel (100) angeordnet ist, die Verstellung des Röhrenkopfes (10) hinsichtlich des Zephalostats zu steuern, und/oder das Steuersystemmittel (100) zur Steuerung der durch zephalostatische Photographie erforderlichen Blendenänderung zu verstellen.

**Revendications**

1. Appareil panoramique à rayons X pour la tomographie dentaire, du type permettant d'obtenir des images d'une voûte dentaire, et comprenant:

un bras (11) monté pivotant dans un plan horizontal autour d'un axe vertical ($O_2$);

un mécanisme pour entraîner en rotation ledit arbre (11) qui comporte un moteur à mouvement rotatif (31);

un générateur de rayons X (40) qui comprend un tube à rayons X, possédant une tête de tube (10), ladite tête de tube (10) étant montée à une des extrémités dudit bras (11) afin d'être entraînée en rotation avec celui-ci;

une cartouche (20) dans laquelle est enfermé un film sensible aux rayons X (RF) ainsi qu'un moteur de déplacement du film (21) qui est monté à l'autre extrémité dudit bras (11) afin d'être entraînée avec celui-ci; et

un mécanisme pour contrôler la vitesse desdits moteurs (21, 31), caractérisé en ce que

l'axe de rotation ($O_2$, $O_3$) dudit bras (11) est guidé avec précision le long d'une trajectoire courbe déterminée mécaniquement, ladite trajectoire étant la même quelles que soient la taille et la forme de la mâchoire que l'on désire photographier,

ledit bras pivote (secteur c) au niveau de l'aire avant de la voûte dentaire et autour dudit axe vertical ($O_2$) déterminé, et avant et après ladite rotation (secteur c) déterminée, le centre de rotation dudit bras (11) se déplace avec précision le long de ladite trajectoire incurvée (secteur d) autour d'un axe vertical ($O_3$) prédéterminé, alors qu'au niveau des aires de l'extrémité de la voûte dentaire, l'axe vertical de rotation décrit de plus, à distance le long de la trajectoire incurvée (secteur e) depuis l'axe de rotation ($a_0$) de la mâchoire (J) en dessous de la photographie,

le mécanisme de contrôle (100) est arrangé afin de pourvoir ajuster la vitesse ($v_d$) dudit moteur à mouvement rotatif (31) et est arrangé pour maintenir la vitesse ($v_f$) dudit moteur de déplacement du film (21) de façon sensiblement constante afin d'entraîner le film à une vitesse constante de manière que l'emplacement d'une couche de voûte soit photographiée en subissant seulement une variation de la vitesse du moteur à mouvement rotatif (31) durant le temps d'exposition, et

ces opérations étant effectuées par le guidage des données de contrôle du manipulateur et par un programme de travail mémorisé dans ledit mécanisme de contrôle, les voûtes dentaires de tailles et de formes variables étant photographiées par ledit appareil panoramique à rayons X pour la tomographie à la fois avec précision et durant une durée d'exposition adaptée.

2. Appareil panoramique à rayons X pour la tomographie selon la revendication 1, caractérisé en ce que le mécanisme de contrôle (100) est également capable de contrôler l'ouverture d'un volet primaire automatique (15) et/ou les changements d'ouverture (62) d'un volet.

3. Appareil panoramique à rayons X pour la tomographie selon la revendication 1 ou 2, caractérisé en ce que le mécanisme de contrôle (100) comprend une combinaison entre

un microprocesseur (108) dans lequel est installée le mémoire du programme (106) et la mémoire de travail (105),

un équipement (102, 103) permettent de contrôler le mécanisme, une partie de connexion reliée au bus (104) du microprocesseur (108), avec laquelle la partie de connexion du microprocesseur (108) est arrangée afin de pouvoir effectuer le contrôle des directions de rotation et des distributions de vitesse du moteur rotatif (31) du bras (11) de l'appareil et du moteur de déplacement du film (21).

4. Appareil panoramique à rayons X pour la tomographie selon la revendication 3, caractérisé en ce que lesdites parties de connexion sont capables de contrôler en plus des moteurs (21, 31) un autre moteur (14) du volet primaire (15) par l'intermédiaire de contrôleurs électroniques (112).

5. Appareil panoramique à rayons X pour la tomographie selon la revendication 3 ou 4, caractérisé en ce que lesdites parties de connexion du microprocesseur (108) sont également capables de contrôler le générateur de rayons X (40) servant à alimenter la tête du tube (10) et en ce que le microprocesseur (108) est arrangé pour admettre en entrée des informations relatives à l'état de l'extrémité du tube (10) par l'intermédiaire de la partie de connexion.

6. Appareil panoramique à rayons X pour la tomographie selon l'une des revendications 1 à 5, caractérisé en ce que le mécanisme de contrôle (100) est programmé pour contrôler à la fois la distribution de vitesse (G) du moteur rotatif (31) dudit bras (11) ainsi que la distribution de vitesse (H) du moteur de déplacement du film (21), indépendamment l'une de l'autre.

7. Appareil panoramique à rayons X pour la tomographie selon l'une des revendications 1 à 6, caractérisé en ce que les photographies normales sont effectuées sous une tension d'anode constante et/ou sous un courant d'anode constant dans le générateur de rayons X (40).

8. Appareil panoramique à rayons X pour la tomographie selon l'une des revendications 1 à 6, caractérisé en ce que le contrôle de la tension de l'anode du générateur de rayons X (40) permet à certaines aires seulement de la voûte dentaire d'être photographiées.

9. Appareil panoramique à rayons X pour la tomographie selon l'une des revendications 1 à 8, caractérisé en ce que le taux d'irradiation du patient fst minimisé grâce au fait que le volet primaire (14, 15) est contrôlé par le mécanisme de contrôle (100) lorsqu'il n'est pas nécessaire de photographier le patient sur toute la hauteur du cliché.

10. Appareil panoramique à rayons X pour la tomographie selon l'une des revendications 1 à 9, caractérisé en ce que le mécanisme est programmé pour photographier seulement de petites surfaces du patient (P) telles que les régions des glandes salivaires ou les régions des sinus maxillaires.

11. Appareil panoramique à rayons X pour la tomographie selon l'une des revendications 1 à 10, caractérisé en ce que le mécanisme de contrôle (100) est arrangé pour être capable de contrôler le courant d'anode, la tension d'anode et/ou la durée d'exposition au tube à rayons X, et ce, en fonction des nécessités du cliché céphalostatique.

12. Appareil panoramique à rayons X pour la tomographie selon la revendication 11, caractérisé en ce que le mécanisme de contrôle (100) est arrangé pour pouvoir contrôler le réglage de la tête du tube (10) par rapport au céphalostat et/ou le mécanisme de contrôle (100) est réglé de façon à contrôler les variations du fonctionnement du volet requises pour le cliché céphalostatique.

**FIG.1**

CONTROL SYSTEM — 100

FIG.2

EP 0 215 757 B1

FIG. 3

FIG.4

FIG.5

FIG.7

FIG.6

FIG. 9

FIG. 10

FIG. 8

FIG. 11

EP 0 215 757 B1